# EUROPEAN PATENT SPECIFICATION

(11) **EP 3 748 343 B1**
(45) Date of publication and mention of the grant of the patent: **17.08.2022**
(21) Application number: 19178694.6
(22) Date of filing: 06.06.2019
(51) Int. Cl.: G01N 21/89, G01N 33/36

(54) **OPTICAL SENSOR DEVICE FOR DETECTING FOREIGN MATERIAL IN AN ELONGATE TEXTILE BODY**
OPTISCHE SENSORVORRICHTUNG ZUR DETEKTION VON FREMDSTOFFEN IN EINEM LÄNGLICHEN TEXTILEN KÖRPER
DISPOSITIF DE CAPTEUR OPTIQUE POUR LA DÉTECTION DE MATÉRIAU ÉTRANGER DANS UN CORPS TEXTILE ALLONGÉ

(43) Date of publication of application: 09.12.2020
(73) Proprietor: Gebrüder Loepfe AG, 8623 Wetzikon (CH)
(72) Inventor: KNECHTLE, Stefan, 8623 Wetzikon (CH); KHIAR, Amir, 8400 Winterthur (CH)
(74) Representative: Sutter, Kurt

(56) References cited:
- EP-A1- 3 130 553
- WO-A2-2014/089713
- DE-A1- 1 623 331
- US-A- 5 768 938

## Description

### Technical Field

The invention relates to an optical sensor device for detecting foreign material in an elongate body, in particular an elongate textile body, such as a yarn. This type of sensor is e.g. used in quality control and monitoring for detecting contaminants in the elongate body, such as foreign fibers or vegetable matter.

### Background Art

US 5414520 and EP 1508797 describe optical sensor devices for measuring foreign material. They propose using two light sources and three light detectors arranged around the textile body in order to detect foreign material in a yarn. At least one of the detectors is used for measuring transmitted light in order to estimate the thickness of the yarn while at least one other detector is used to detect light reflected from the yarn. Since the reflected light depends on both the thickness as well as the presence of contaminations, suitable processing allows to reduce the thickness dependence of the signals.

US 5 768 938 describes a sensor device having a support body forming a measurement slit and light sources and light detectors alternatingly arranged at different angles around it.

### Disclosure of the Invention

The problem to be solved by the present invention is to provide a sensor device of this type with improved reliability that is easy to assemble.

This problem is solved by the sensor device of claim 1. Accordingly, the optical sensor device for detecting foreign material in an elongate body, in particular an elongate textile body, comprises a measurement chamber for receiving the elongate body. The sensor device further comprises a plurality of light sources as well as a plurality of light detectors. According to the invention, there are:
- at least one light detector arranged between at least two light sources on a first side of the measurement chamber and
- at least one light source arranged between at least two light detectors on a second side of the measurement chamber.

The second side of the measurement chamber is opposite to the first side of the measurement chamber.

This design makes the signal of the device less dependent on the body's position. If the body is offset from the center of the measurement chamber, the distance to some of the light sources and light detectors increases while the distance to others decreases, which leads to an at least partial compensation of the effects of such a displacement.

In this context, the "center of the measurement chamber" corresponds to the nominal position of the elongate body during the measurement.

In one embodiment, at least three of the light sources and at least three of the light detectors are alternatingly arranged around said measurement chamber.

Advantageously, the number of light sources and the number of light detectors are equal, so all of them can be alternatingly arranged at different angular positions. In this context, several light emitters located in a common housing (such as several light emitters with differing spectral emission spectra) or close to each other (in particular closer than 3 mm) and/or several light sensors in a common housing (such as several light sensors having differing spectral sensitivities) or close to each other (in particular closer than 3 mm) count as one light source and one light detector, respectively.

In particular, the number of light sources and the number of light detectors are odd numbers. This is particularly advantageous if least one of the light detectors can be arranged opposite from one of the light sources and vice versa, which allows to measure the transmission through the measuring region. In particular, each light detector is arranged opposite from one of the light sources and vice versa.

According to the invention, the sensor device comprises a support body having:
- Opposing first and second sides as well as peripheral sides extending transversally to the first and second sides.
- A slit open at the first side and the second side: This slit forms the measurement chamber for the elongate body. The openings at the first and second sides allow the elongate body to enter and leave the measurement chamber.
- Radial light channels extending between
said measurement chamber and said peripheral sides: These light channels allow light to pass from the light sources to the measurement chamber and from the measurement chamber to the light detectors.

The light sources and the light detectors are alternatingly arranged at the light channels at the peripheral sides. This design allows to position the various elements using a single body. Also, the light sources and light detectors can easily be mounted to the peripheral sides of the carrier body.

The sensor device has a first carrier plate on a first peripheral side of the support body and a second carrier plate on a second peripheral side of said support body, with the second peripheral side being opposite first peripheral side. This allows to mount the light source(s) and light detector(s) on the first peripheral side to the first carrier plate and the light source(s) and the light detector(s) on the second peripheral side to the second carrier plate.

The carrier plates may be printed circuit boards comprising the electrical leads to connect to the light sources and light detectors.

The carrier plates are advantageously mounted to the support body for easy assembly.

The carrier plates may be parallel to each other.

The sensor device is particularly suited for measuring foreign material in an elongate textile body, such as a yarn. In one advantageous application, the sensor device is used in a yarn clearer.

Other advantageous embodiments are listed in the dependent claims as well as in the description below.

### Brief Description of the Drawings

The invention will be better understood and objects other than those set forth above will become apparent from the following detailed description thereof. Such description refers to the annexed drawings, wherein:
Fig. 1 shows a schematic view of a sensor device,
Fig. 2 shows a sectional view along line II-II of Fig. 1,
Fig. 3 shows another example of a sensor device, and
Fig. 4 is a sectional view along line IV-IV of Fig. 3.

### Modes for Carrying Out the Invention

### Embodiment 1

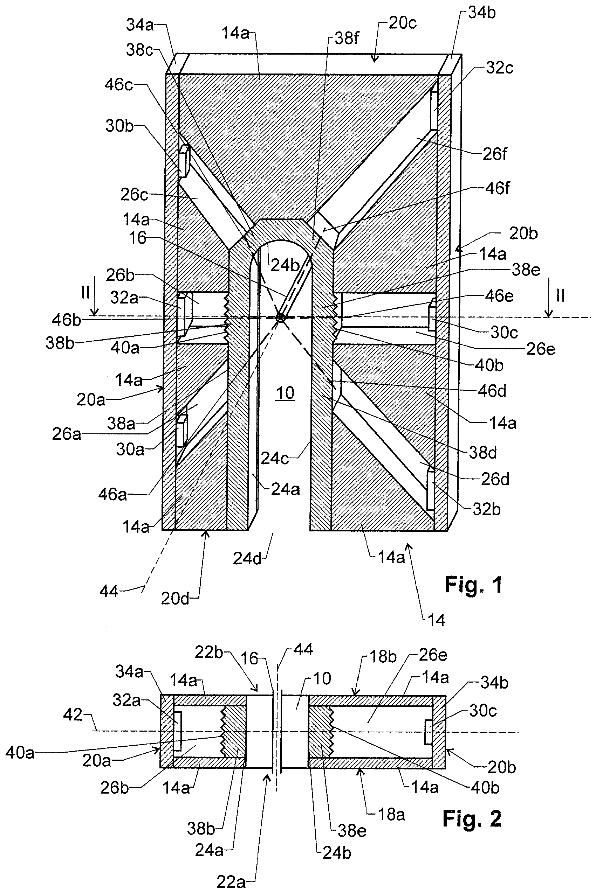

Figs. 1 and 2 show a schematic representation of a sensor device cut along its middle.

The device comprises a slit-shaped measurement chamber 10 formed by a slit in a support body 14. Measuring chamber 10 is designed to receive the elongate object 16.

Support body 14 has a first side 18a, a second side 18b, which are advantageously parallel to each other (see Fig. 2). It further has peripheral sides 20a, 20b, 20c, and 20d (see Figs. 1 and 2) extending transversally, in particular perpendicularly, to first and second side 18a, 18b.

Measurement chamber 10 is open at first side 18a and second side 18b, where it forms openings 22a, 22b through which elongate object 16 can enter and exit during the measurement.

At its sides perpendicular to the first and second side 18a, 18b, measurement chamber 10 is closed on three sides 24a, 24b, and 24c by support body 14 while it is open at a fourth side 24d for inserting elongate body 16 before operation.

Radial light channels 26a - 26f extend through support body 14 between measurement chamber 10 and a first peripheral side 20a and a second peripheral side 20b.

Advantageously, first peripheral side 20a and second peripheral side 20b are located opposite to each other, and in particular they are parallel to each other.

The sensor device further comprises several light sources 30a, 30b, 30c and several light detectors 32a, 32b, 32c. In the present embodiment, there are three light sources as well as three light detectors. As mentioned above, that number may be larger.

The light sources 30a, 30b, 30c and the light detectors 32a, 32b, 32c are alternatingly arranged at the light channels 26a - 26f, namely at the first and second peripheral sides 20a, 20b of support body 14.

The light sources 30a, 30b, 30c and the light detectors 32a, 32b, 32c are mounted on first and second carrier plates 34a, 34b.

First carrier plate 34a is mounted to first peripheral side 20a of support body 14. It carries the light sources 30a, 30b as well as the light detector 32a arranged on that side. Second carrier plate 34b is mounted to second peripheral side 20b of support body 14. It carries the light source 30c as well as the light detectors 32b and 32c arranged on that side.

The carrier plates 34a, 34b are advantageously printed circuit boards and they are arranged parallel to each other. They may be mechanically connected to support body 14 in defined spatial relation such that the light sources and light detectors are located at the desired positions in the light channels 26a - 26f.

As can be seen, there is a first light source 30a and a second light source 30b as well as a first light detector 32a arranged on the first side 20a, and there is a third light source 30c as well as a second light detector 32b and a third light detector 32c arranged on the second side.

The first light source 30a is opposite the third light detector 32c, the second light source 30b is opposite the second light detector 32b, and the third light source 30c is opposite the first light detector 32a. In this context, "opposite" means on opposing sides of the nominal position of elongate body 16 in measurement chamber 10 (i.e. the center of measurement chamber 10) .

In more general terms, each light detector is arranged opposite from one of the light sources and vice versa.

As mentioned above, at least one light detector 32a is arranged between at least two light sources 30a, 30b on a first side of measurement chamber 10, and at least one light source 30c is arranged between at least two light detectors 32b, 32c on a second side of measurement chamber 10. Using the Helmholtz reciprocity principle, it can be shown that such an arrangement leads to a homogeneous measurement chamber where small deviations of the position of elongate body 16 from its nominal operating position have only a weak influence on the measured signals.

As can be seen, there is a larger number of light sources on first peripheral side 20a than on second peripheral side 20b. In the present embodiment, there are two light sources 30a and 30b on first peripheral side 20a and only one light source 30c on second peripheral side 20b. In contrast to this, there is a larger number of light detectors on second peripheral side 20b than on first peripheral side 20a. In the present embodiment, there are two light detectors 32b and 32c on second peripheral side 20b and only one light detector 32a on first peripheral side 20a.

Typically, the light detectors 32a - 32c have a larger active (i.e. light-sensitive) area than the active (i.e. light-emitting) area of the light sources 30a - 30c. The Helmholtz reciprocity principle, as mentioned above, is based on the assumption that the light sources and light detectors have equal active area. To compensate for this at least in part, support body 14 is designed such that first peripheral side 20a (and the light sources and light detectors arranged there) is closer to measurement chamber 10 than second peripheral side 20b (and the light sources and light detectors arranged there).

The shown embodiment has a first light channel 26a extending between first light source 30a and measurement chamber 10, a second light channel 26b extending between first light detector 32a and measurement chamber 10, a third light channel 26c extending between second light source 30b and measurement chamber 10, a fourth light channel 26d extending between second light detector 32b and measurement chamber 10, an fourth light channel 26e extending between third light source 30c and measurement chamber 10, and a sixth light channel 26f extending between third light detector 32c and measurement chamber 10.

Hence, the first, second, and third light channel 26a - 26c extend through support body 14 between measurement chamber 10 and first peripheral side 20a. The fourth, fifth, and sixth light channel 26d - 26f extend through support body 14 between measurement chamber 10 and second peripheral side 20b.

The second light channel 26b is arranged between the first light channel 26a and the third light channel 26c. Similarly, the fourth light channel 26e is arranged between the fourth light channel 26d and the sixth light channel 26e.

The second light channel 26b and the fifth light channel 26e are coaxial, such that light source 30c is arranged exactly vis-à-vis light detector 32a in order to provide an accurate transmission measurement of measurement chamber 10. This allows to generate a signal dependent on the thickness of elongate object 16.

Support body 14 comprises a first body element 14a and a second body element 14b.

First body element 14a is advantageously of a non-transparent material (i.e. a material not transparent in the spectral region of overlap between the emission of the light sources and the sensitivity of the light detectors), and it separates the light channels from each other in order to suppress optical crosstalk between them.

In more general terms, the light channels 26a - 26f (optically) advantageously communicate through the measurement chamber, i.e. a significant part of the light (i.e. at least 10%, in particular at least 25%) passing from one to the other has passed through measurement chamber 10.

Second body element 14b is of a transparent material (i.e. a material transparent in the spectral region over overlap between the emission of the light sources and the sensitivity of the light detectors). It forms transparent windows 38a - 38f between the light channels 26a - 26f and measurement chamber 10. These windows prevent dirt from entering the light channels 26a - 26f. Advantageously, the windows 38a - 38f are transparent in the sense that they let at least 50% of the light in the spectrum of overlap between the light sources and the light detectors pass.

The windows 38b and 38e form a first diffusor 40a and a second diffusor 40b arranged at the interface between second light channel 26b and measurement chamber 10 as well as at the interface between fourth light channel 26e and measurement chamber 10. They increase the accuracy of the measurement.

Diffusors 40a, 40b may e.g. be formed by small structures (having sizes between 10 and 500 µm) arranged on a surface of the windows 38b, 38e, in particular on a side of these windows that faces away from measurement chamber 10.

Advantageously, first body element 14a and second body element 14b are manufactured by two-component casting or two-component moulding in order to form support body 14.

The light channels 26a - 26f are advantageously hollow, i.e. filled with a gas, in particular air. In order to increase their light ducting properties, their inner surfaces are advantageously smooth and reflective.

Alternatively, the light channels 26a - 26f may be filled with a transparent solid.

As can be seen from Fig. 2, the light sources 30a - 30c, the light detectors 32a - 32c, and the measurement chamber 10 are arranged in a common plane 42 (i.e. they are intersected by said common plane). Advantageously, this common plane 42 extends parallel to first side 18a and second side 18b of support body 14 and/or perpendicular to the longitudinal axis 44 of elongate body 16.

In said common plane 42, the light sources 30a - 30c and the light detectors 32a - 32c are arranged at different angular positions. These angular positions are defined by the connecting lines between the nominal position of the elongate body 16 (i.e. the center of measurement chamber 10) and the respective light source or light detector.

Further, the windows 38a - 38f are arranged at different angular positions 46a - 46f around the center of measurement chamber 10. The angular positions 46a - 46f should be distributed more or less evenly around measuring chamber 10. In particular, the mutual angles between neighbouring ones of the angular positions are advantageously at least 30°.

### Embodiment 2

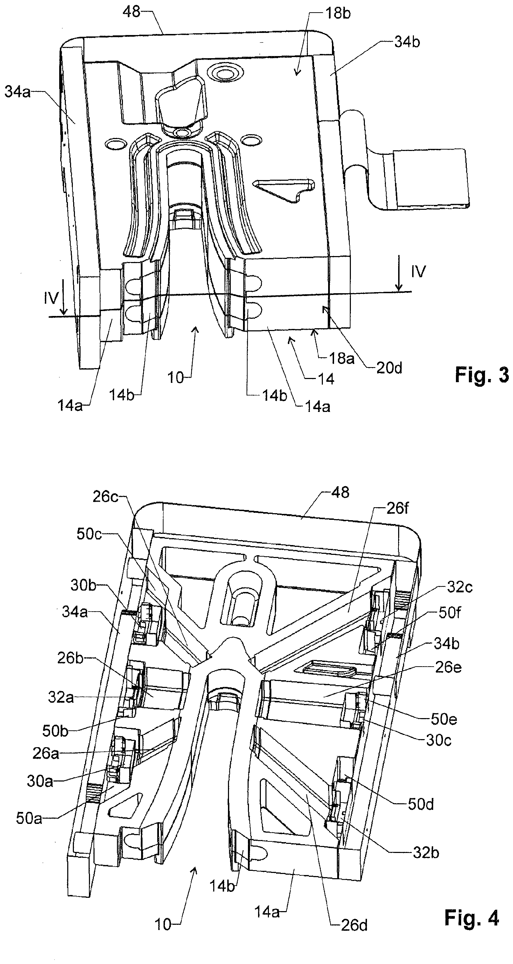

Figs. 3 and 4 show a specific embodiment of the sensor device.

As can be seen, measurement chamber 10 is formed by a slit of support body 14 that widens towards fourth peripheral side 20d.

Transparent second body element 14b forms part of the inner wall of the slit. Non-transparent, first body element 14a forms the first side 18a and the second side 18b of support body 14. It closes the light channels 26a - 26f at these first and second sides 18a, 18b.

The two carrier plates 34a, 34b are mounted to opposite peripheral sides of support body 14 and may e.g. be electrically interconnected by means of a flexible printed circuit 48.

At least some of the light sources 30a - 30c and/or at least some of the light detectors 32a - 32b are located in recesses 50a - 50f of support body 14, with each recess 50a - 50f forming an end of one light channel 26a - 26f.

### Notes

As mentioned, each light source 30a - 30c may comprise several light emitters, such as several light emitters at different wavelengths, in order to perform measurements at different spectral regions. Similarly, each light detector 32a - 32c may comprise several light sensors sensitive to different spectral regions.

The light sources 30a - 30c may comprise LEDs. For example, each light source may comprise several LEDs of different colours.

The light detectors 32a - 32c may e.g. comprise photodiodes.

In the above embodiments, the light sources 30a - 30c and light detectors 32a - 32c are arranged on the first and second peripheral sides 20a, 20b of support body 14 only, which allows to mount them to only two carrier plates 34a, 34b. Alternatively, though, some of them may e.g. also be arranged on third and/or fourth peripheral side 20c, 20d.

While there are shown and described presently preferred embodiments of the invention, it is to be distinctly understood that the invention is not limited thereto but may be otherwise variously embodied and practiced within the scope of the following claims.

## Claims

1. An optical sensor device for detecting foreign material in an elongate body, in particular an elongate textile body, comprising
a measurement chamber (10) for the elongate textile body,
a plurality of light sources (30a - 30c), and
a plurality of light detectors (32a - 32c),
a support body (14) having
- opposing first and second sides (18a, 18b) as well as peripheral sides (20a - 20d) extending transversally to said first and second sides (18a, 18b),
- a slit forming said measurement chamber (10) and being open at the first and second sides (18a, 18b), and
- light channels (26a - 26f) extending between said measurement chamber (10) and said peripheral sides (20a - 20d),
wherein there are
- at least one light detector (32a) arranged between at least two light sources (30a, 30b) on a first side of said measurement chamber (10) and
- at least one light source (30c) arranged between at least two light detectors (32b, 32c) on a second side of said measurement chamber (10),
wherein said second side of said measurement chamber (10) is opposite to said first side of said measurement chamber (10),
wherein said light sources (30a - 30c) and said light detectors (32a - 32c) are alternatingly arranged at said light channels (26a - 26f) at said peripheral sides (20a - 20d)
said optical sensor device being **characterized by** a first carrier plate (34a) on a first peripheral side (20a) of said support body (14) and a second carrier plate (34b) on a second peripheral side (20b) of said support body (14), wherein said second peripheral side (20b) is opposite said first peripheral side (20a),
wherein the light source(s) and light detector(s) (30a, 30b, 32a) on said first peripheral side (20a) are mounted to said first carrier plate (34a) and the light source(s) and the light detector(s) (30c, 32b, 32c) on said second peripheral side (20b) are mounted to said second carrier plate (34b).

2. The optical sensor device of claim 1 wherein a number of said light sources (30a - 30c) is equal to a number of said light detectors (32a - 32c).

3. The optical sensor device of claim 2 wherein said numbers are odd numbers.

4. The optical sensor device of any of the preceding claims wherein at least one of the light detectors (32a - 32c) is arranged opposite from one of the light sources (30a - 30c) and vice versa,
and in particular wherein each light detector is arranged opposite from one of the light sources (30a - 30c) and vice versa.

5. The optical sensor device of any of the preceding claims wherein said carrier plates (34a, 34b)
are printed circuit boards and/or
are mounted to the support body (14) and/or
are parallel to each other.

6. The optical sensor device of any of the preceding claims wherein there is a larger number of said light sources (30a - 30c) on said first peripheral side (20a) and a larger number of light detectors (32a - 32c) on said second peripheral side (20b), wherein said light detectors (32a - 32c) have larger active areas than said light sources (30a - 30c), and wherein said first peripheral side (20a) is closer to said measurement chamber (10) than said second peripheral side (20b).

7. The optical sensor device of any of the preceding claims having
a first, a second, and a third light channel (26a - 26c) extending to said first peripheral side (20a), with said second light channel (26b) being arranged between said first and third light channels (26a, 26c),
a fourth, a fifth, and a sixth light channel (26d - 26f) extending to said second peripheral side (20b), with said fifth light channel (26e) being arranged between said fourth and said sixth light channels (26d, 26f) .

8. The optical sensor device of claim 7 wherein said second and said fifth channels (26b, 26e) are coaxial.

9. The optical sensor device of any of the claims 7 or 8 further comprising a first optical diffusor (40a) arranged at an interface between said second light channel (26b) and said measurement chamber (10) and/or a second optical diffusor (40b) arranged at an interface between said fifth light channel (26e) and said measurement chamber (10).

10. The optical sensor device of any of the preceding claims having a first and a second light source (30a, 30b) and a first light detector (32a) on said first peripheral side (20a) and a third light source (30c) and a second and a third light detector (32b, 32c) on said second peripheral side (20b), wherein
said first light source (30a) is opposite said third light detector (32c),
said second light source (30b) is opposite said second light detector (32b), and
said third light source (30c) is opposite said first light detector (32a).

11. The optical sensor device of any of the claims 4 to 10 wherein said light channels (26a - 26f) communicate through said measurement chamber (10).

12. The optical sensor device of any of the preceding claims further comprising transparent windows (38a - 38f) between said light channels (26a - 26f) and said measurement chamber (10),
and in particular wherein said support body (14) comprises a non-transparent first body element (14a) and a transparent second body element (14b), wherein said first body element (14a) separates said light channels (26a - 26f) and said second body element (14b) forms said windows (38a - 38f).

13. The optical sensor device of claim 12 wherein said windows (38a - 38f) are arranged at different angular positions (46a - 46f) around a center of said measurement chamber (10), wherein all mutual angles between neighbouring ones of said angular positions (46a - 46f) are at least 30°.

14. The optical sensor device of any of the preceding claims wherein said light channels (26a - 26f) are hollow.

15. The optical sensor of any of the preceding claims wherein said measuring chamber (10), said light sources (30a - 30c), and light detectors (32a - 32c) are arranged in a common plane (42).

16. The optical sensor device of any of the preceding claims wherein at least three of said light sources (30a - 30c) and at least three of said light detectors (32a - 32c) are alternatingly arranged around said measurement chamber (10).

## Patentansprüche

1. Eine optische Sensorvorrichtung zum Erfassen von Fremdmaterial in einem langgestreckten Körper, insbesondere einem langgestreckten Textilkörper, umfassend
eine Messkammer (10) für den langgestreckten textilen Körper,
eine Vielzahl von Lichtquellen (30a - 30c), und
eine Vielzahl von Lichtdetektoren (32a - 32c),
einen Trägerkörper (14) mit
- gegenüberliegende erste und zweite Seiten (18a, 18b) sowie peripheren Seiten (20a - 20d), die sich quer zu den ersten und zweiten Seiten (18a, 18b) erstrecken,
- einen Schlitz, der die Messkammer (10) bildet und an den ersten und zweiten Seiten (18a, 18b) offen ist, und
- Lichtkanäle (26a - 26f), die sich zwischen der Messkammer (10) und den peripheren Seiten (20a - 20d) erstrecken,
wobei vorhanden sind
- mindestens ein Lichtdetektor (32a) der zwischen mindestens zwei Lichtquellen (30a, 30b) auf einer ersten Seite der Messkammer (10) angeordnet ist und
- mindestens eine Lichtquelle (30c), die zwischen mindestens zwei Lichtdetektoren (32b, 32c) auf einer zweiten Seite der Messkammer (10) angeordnet ist,
wobei die zweite Seite der Messkammer (10) der ersten Seite der Messkammer (10) gegenüber liegt,
wobei die Lichtquellen (30a - 30c) und die Lichtdetektoren (32a - 32c) abwechselnd an den Lichtkanälen (26a - 26f) an den peripheren Seiten (20a - 20d) angeordnet sind
wobei die optische Sensorvorrichtung **gekennzeichnet ist durch** eine erste Trägerplatte (34a) auf einer ersten peripheren Seite (20a) des Trägerkörpers (14) und eine zweite Trägerplatte (34b) auf einer zweiten peripheren Seite (20b) des Trägerkörpers (14), wobei die zweite periphere Seite (20b) der ersten peripheren Seite (20a) gegenüberliegt,
wobei die Lichtquelle(n) und der/die Lichtdetektor(en) (30a, 30b, 32a) auf der ersten peripheren Seite (20a) an der ersten Trägerplatte (34a) angebracht sind und die Lichtquelle(n) und der/die Lichtdetektor(en) (30c, 32b, 32c) auf der zweiten peripheren Seite (20b) an der zweiten Trägerplatte (34b) angebracht sind.

2. Die optische Sensorvorrichtung nach Anspruch 1, wobei die Anzahl der Lichtquellen (30a - 30c) gleich der Anzahl der Lichtdetektoren (32a - 32c) ist.

3. Die optische Sensorvorrichtung nach Anspruch 2, wobei die Anzahl ungerade ist.

4. Die optische Sensorvorrichtung nach einem der vorangehenden Ansprüche, wobei mindestens einer der Lichtdetektoren (32a - 32c) gegenüber einer der Lichtquellen (30a - 30c) angeordnet ist und umgekehrt,
und insbesondere wobei jeder Lichtdetektor gegenüber einer der Lichtquellen (30a - 30c) und umgekehrt angeordnet ist.

5. Die optische Sensorvorrichtung nach einem der vorangehenden Ansprüche, wobei die Trägerplatten (34a, 34b)
gedruckte Leiterplatten sind und/oder
am Trägerkörper (14) befestigt sind und/oder
parallel zueinander sind.

6. Die optische Sensorvorrichtung nach einem der vorangehenden Ansprüche, wobei eine grössere Anzahl der Lichtquellen (30a - 30c) auf der ersten peripheren Seite (20a) und eine grössere Anzahl von Lichtdetektoren (32a - 32c) auf der zweiten peripheren Seite (20b) vorhanden ist, wobei die Lichtdetektoren (32a - 32c) grössere aktive Flächen als die Lichtquellen (30a - 30c) aufweisen und wobei die erste periphere Seite (20a) näher an der Messkammer (10) ist als die zweite periphere Seite (20b).

7. Die optische Sensorvorrichtung nach einem der vorangehenden Ansprüche mit
einem ersten, einem zweiten und einem dritten Lichtkanal (26a - 26c), die sich zu der ersten peripheren Seite (20a) erstrecken, wobei der zweite Lichtkanal (26b) zwischen dem ersten und dem dritten Lichtkanal (26a, 26c) angeordnet ist,
einen vierten, einen fünften und einen sechsten Lichtkanal (26d - 26f), die sich zu der zweiten peripheren Seite (20b) erstrecken, wobei der fünfte Lichtkanal (26e) zwischen dem vierten und dem sechsten Lichtkanal (26d, 26f) angeordnet ist.

8. Die optische Sensorvorrichtung nach Anspruch 7, wobei der zweite und der fünfte Kanal (26b, 26e) koaxial sind.

9. Die optische Sensorvorrichtung nach einem der Ansprüche 7 oder 8 weiter umfassend einen ersten optischen Diffusor (40a), der an einer Schnittstelle zwischen dem zweiten Lichtkanal (26b) und der Messkammer (10) angeordnet ist, und/oder einen zweiten optischen Diffusor (40b), der an einer Schnittstelle zwischen dem fünften Lichtkanal (26e) und der Messkammer (10) angeordnet ist.

10. Die optische Sensorvorrichtung nach einem der vorangehenden Ansprüche mit einer ersten und einer zweiten Lichtquelle (30a, 30b) und einem ersten Lichtdetektor (32a) auf der ersten peripheren Seite (20a) und einer dritten Lichtquelle (30c) und einem zweiten und einem dritten Lichtdetektor (32b, 32c) auf der zweiten peripheren Seite (20b), wobei
die erste Lichtquelle (30a) sich gegenüber dem dritten Lichtdetektor (32c) befindet,
die zweite Lichtquelle (30b) sich gegenüber dem zweiten Lichtdetektor (32b) befindet, und
die dritte Lichtquelle (30c) dem ersten Lichtdetektor (32a) gegenüberliegt.

11. Die optische Sensorvorrichtung nach einem der Ansprüche 4 bis 10, wobei die Lichtkanäle (26a - 26f) durch die Messkammer (10) kommunizieren.

12. Die optische Sensorvorrichtung nach einem der vorangehenden Ansprüche weiter umfassend transparente Fenster (38a - 38f) zwischen den Lichtkanälen (26a - 26f) und der Messkammer (10),
und insbesondere wobei der Trägerkörper (14) ein nicht-transparentes erstes Körperelement (14a) und ein transparentes zweites Körperelement (14b) umfasst, wobei das erste Körperelement (14a) die Lichtkanäle (26a - 26f) trennt und das zweite Körperelement (14b) die Fenster (38a - 38f) bildet.

13. Die optische Sensorvorrichtung nach Anspruch 12, wobei die Fenster (38a - 38f) in verschiedenen Winkelpositionen (46a - 46f) um ein Zentrum der Messkammer (10) angeordnet sind, wobei alle gegenseitigen Winkel zwischen benachbarten Winkelpositionen (46a - 46f) mindestens 30° betragen.

14. Die optische Sensorvorrichtung nach einem der vorangehenden Ansprüche, wobei die Lichtkanäle (26a - 26f) hohl sind.

15. Der optischer Sensor nach einem der vorangehenden Ansprüche, wobei die Messkammer (10), die Lichtquellen (30a - 30c) und die Lichtdetektoren (32a - 32c) in einer gemeinsamen Ebene (42) angeordnet sind.

16. Die optische Sensorvorrichtung nach einem der vorangehenden Ansprüche, wobei mindestens drei der Lichtquellen (30a - 30c) und mindestens drei der Lichtdetektoren (32a - 32c) abwechselnd um die Messkammer (10) herum angeordnet sind.

## Revendications

1. Un dispositif de détection optique pour détecter une matière étrangère dans un corps allongé, en particulier un corps textile allongé, comprenant
une chambre de mesure (10) pour le corps textile allongé,
une pluralité de sources de lumière (30a - 30c), et
une pluralité de détecteurs de lumière (32a - 32c),
un corps de support (14) ayant
- des premier et deuxième côtés opposés (18a, 18b) et des côtés périphériques (20a - 20d) qui s'étendent transversalement par rapport aux premier et deuxième côtés (18a, 18b),
- une fente formant la chambre de mesure (10) et ouverte sur les premier et deuxième côtés (18a, 18b), et
- des canaux de lumière (26a - 26f) qui s'étendent entre la chambre de mesure (10) et les côtés périphériques (20a - 20d),
où sont présents
- au moins un détecteur de lumière (32a) disposé entre au moins deux sources de lumière (30a, 30b) sur un premier côté de la chambre de mesure (10) et
- au moins une source de lumière (30c) disposée entre au moins deux détecteurs de lumière (32b, 32c) sur un deuxième côté de la chambre de mesure (10),
le deuxième côté de la chambre de mesure (10) étant opposé au premier côté de la chambre de mesure (10),
dans lequel les sources de lumière (30a - 30c) et les détecteurs de lumière (32a - 32c) sont disposés en alternance sur les canaux de lumière (26a - 26f) sur les côtés périphériques (20a - 20d)
le dispositif de détection optique étant **caractérisé par** une première plaque de support (34a) sur un premier côté périphérique (20a) du corps de support (14) et une seconde plaque de support (34b) sur un second côté périphérique (20b) du corps de support (14), le second côté périphérique (20b) étant opposé au premier côté périphérique (20a),
la ou les sources de lumière et le ou les détecteurs de lumière (30a, 30b, 32a) sur la première face périphérique (20a) étant montés sur la première plaque de support (34a) et la ou les sources de lumière et le ou les détecteurs de lumière (30c, 32b, 32c) sur la deuxième face périphérique (20b) étant montés sur la deuxième plaque de support (34b).

2. Le dispositif de détection optique selon la revendication 1, dans lequel le nombre de sources de lumière (30a - 30c) est égal au nombre de détecteurs de lumière (32a - 32c).

3. Le dispositif de détection optique selon la revendication 2, dans lequel les nombres sont impairs.

4. Le dispositif de détection optique selon l'une des revendications précédentes, dans lequel au moins un des détecteurs de lumière (32a - 32c) est disposé de manière opposée par rapport à une des sources de lumière (30a - 30c) et inversement,
et particulièrement dans lequel chaque détecteur de lumière est disposé de manière opposée par rapport à une des sources de lumière (30a - 30c) et inversement.

5. Le dispositif de détection optique selon l'une des revendications précédentes, dans lequel les plaques support (34a, 34b)
sont des cartes de circuits imprimés et/ou sont fixées au corps de support (14) et/ou sont parallèles entre elles.

6. Le dispositif de détection optique selon l'une des revendications précédentes, dans lequel un plus grand nombre des sources de lumière (30a - 30c) est présent sur le premier côté périphérique (20a) et un plus grand nombre de détecteurs de lumière (32a - 32c) est présent sur le deuxième côté périphérique (20b), dans lequel les détecteurs de lumière (32a - 32c) présentent des surfaces actives plus grandes que les sources de lumière (30a - 30c) et dans lequel le premier côté périphérique (20a) est plus proche de la chambre de mesure (10) que le deuxième côté périphérique (20b).

7. Le dispositif de détection optique selon l'une des revendications précédentes, comprenant
un premier, un deuxième et un troisième canal de lumière (26a - 26c) s'étendant vers le premier côté périphérique (20a), le deuxième canal de lumière (26b) étant situé entre le premier et le troisième canal de lumière (26a, 26c),
un quatrième, un cinquième et un sixième canal de lumière (26d - 26f) s'étendant vers le deuxième côté périphérique (20b), le cinquième canal de lumière (26e) étant disposé entre le quatrième et le sixième canal de lumière (26d, 26f).

8. Le dispositif de détection optique selon la revendication 7, dans lequel le deuxième et le cinquième canal (26b, 26e) sont coaxiaux.

9. Le dispositif de détection optique selon l'une des revendications 7 ou 8 comprenant en outre un premier diffuseur optique (40a) disposé à une interface entre le deuxième canal de lumière (26b) et la chambre de mesure (10), et/ou un deuxième diffuseur optique (40b) disposé à une interface entre le cinquième canal de lumière (26e) et la chambre de mesure (10).

10. Le dispositif de détection optique selon l'une des revendications précédentes ayant une première et une deuxième source de lumière (30a, 30b) et un premier détecteur de lumière (32a) sur le premier côté périphérique (20a) et une troisième source de lumière (30c) et un deuxième et un troisième détecteur de lumière (32b, 32c) sur le deuxième côté périphérique (20b), dans lequel
la première source de lumière (30a) est située en face du troisième détecteur de lumière (32c),
la deuxième source de lumière (30b) est située en face du deuxième détecteur de lumière (32b), et
la troisième source lumineuse (30c) est située en face du premier détecteur de lumière (32a).

11. Le dispositif de détection optique selon l'une des revendications 4 à 10, dans lequel les canaux de lumière (26a - 26f) communiquent à travers la chambre de mesure (10).

12. Le dispositif de détection optique selon l'une des revendications précédentes comprenant en outre des fenêtres transparentes (38a - 38f) entre les canaux de lumière (26a - 26f) et la chambre de mesure (10),
et particulièrement dans lequel le corps de support (14) comprend un premier élément de corps (14a) non transparent et un deuxième élément de corps (14b) transparent, le premier élément de corps (14a) séparant les canaux de lumière (26a - 26f) et le deuxième élément de corps (14b) formant les fenêtres (38a - 38f).

13. Le dispositif de détection optique selon la revendication 12, dans lequel les fenêtres (38a - 38f) sont disposées dans différentes positions angulaires (46a - 46f) autour d'un centre de la chambre de mesure (10), tous les angles mutuels entre des positions angulaires adjacentes (46a - 46f) étant d'au moins 30°.

14. Le dispositif de détection optique selon l'une des revendications précédentes, dans lequel les canaux de lumière (26a - 26f) sont creux.

15. Le dispositif de détection optique selon l'une des revendications précédentes, dans lequel la chambre de mesure (10), les sources de lumière (30a - 30c) et les détecteurs de lumière (32a - 32c) sont disposés dans un plan commun (42).

16. Le dispositif de détection optique selon l'une des revendications précédentes, dans lequel au moins trois des sources de lumière (30a - 30c) et au moins trois des détecteurs de lumière (32a - 32c) sont disposés alternativement autour de la chambre de mesure (10) .
